# FASCICULE DE BREVET EUROPEEN

(11) **EP 2 321 191 B1**
(45) Date de publication et mention de la délivrance du brevet: **29.10.2014**
(21) Numéro de dépôt: 09786089.4
(22) Date de dépôt: 31.07.2009
(51) Int. Cl.: B65D 47/18, A61F 9/00

(54) **FLACON DE CONDITIONNEMENT DE LIQUIDE A DISTRIBUER GOUTTE A GOUTTE A PROTECTION ANTIBACTERIENNE**
FLASCHE ZUR VERPACKUNG EINER TROPFENWEISE ABZUGEBENDEN FLÜSSIGKEIT MIT ANTIBAKTERIELLEM SCHUTZ
BOTTLE FOR PACKAGING LIQUID THAT IS TO BE DISPENSED DROP BY DROP, WITH ANTIBACTERIAL PROTECTION

(30) Priorité: 31.07.2008 FR 0804420
(43) Date de publication de la demande: 18.05.2011
(73) Titulaire: Laboratoires THEA, 63100 Clermont-Ferrand (FR)
(72) Inventeur: CHIBRET, Jean-Frédéric, F-63000 Clermont Ferrrand (FR); DEFEMME, Alain, F-63400 Chamalieres (FR); FAURIE, Michel, F-63960 Veyre-Monton (FR); MERCIER, Fabrice, F-63000 Clermont-Ferrand (FR)
(74) Mandataire: Thibon, Norbert
(86) Numéro de dépôt international: PCT/IB2009/006420
(87) Numéro de publication internationale: WO 2010/013131

(56) Documents cités:
- WO-A1-02/38464
- WO-A1-2006/043295
- WO-A1-2007/033480
- CA-A1- 2 492 255
- US-A- 5 373 972

## Description

La présente invention concerne la conception et la réalisation d'un flacon de conditionnement de liquide à distribuer goutte à goutte par l'intermédiaire d'une tête de distribution à embout compte-gouttes.

L'invention s'applique à tous les domaines dans lesquels il peut être souhaitable de distribuer un liquide goutte par goutte, notamment pour les produits pharmaceutiques ou les produits cosmétiques, ou pour toute solution exempte d'agents conservateurs anti-microbiens.

Le domaine d'application particulièrement préféré de l'invention, qui sera plus spécifiquement décrit dans la présente description à titre d'exemple, sans être aucunement limitatif, concerne le conditionnement et la distribution des liquides ophtalmiques, qui sont destinés à être appliqués localement dans l'oeil, sous forme de gouttes.

La plupart des solutions ophtalmiques, quelle que soit leur fonction (traitement d'une maladie oculaire, cicatrisation, hydratation, etc.), sont commercialisées contenues dans un réservoir ménagé à l'intérieur d'un flacon de conditionnement qui est équipé d'un embout compte-gouttes pour leur distribution directement dans l'oeil. L'embout est percé d'un canal central d'expulsion du liquide depuis le réservoir jusqu'à l'extérieur. Il se fixe généralement au-dessus du réservoir. Plus exactement, dans les flacons auxquels s'intéresse la présente invention, l'embout fait partie d'une tête de distribution dans laquelle il prolonge une partie interne, ou nacelle, qui s'insère étanche dans un goulot du flacon en assurant la communication entre le réservoir et le canal d'expulsion.

Tous les flacons de ce type, voir par exemple WO0238464, posent un problème de protection contre la prolifération microbienne, qui risque d'entraîner, au moment de la distribution des gouttes, la contamination microbiologique de l'oeil du patient. Pour y remédier, il est classique d'utiliser des agents conservateurs anti-microbiens que l'on introduit en mélange dans la solution. Mais de tels agents, tel que par exemple le chlorure de benzalkonium, ont le grave inconvénient d'être agressifs pour l'oeil.

Dans le même but, il est de plus en plus fréquent d'équiper les systèmes de délivrance de gouttes d'une membrane filtrante anti-bactérienne, interposée sur le trajet du liquide entre le réservoir et l'embout, pour empêcher les contaminations extérieures d'atteindre la solution contenue dans le réservoir. Cette solution est satisfaisante au niveau du contenu du flacon et tant que le flacon reste protégé par un capuchon enfermant l'embout compte-gouttes et par son emballage de commercialisation. Elle ne l'est plus après que la tête de distribution ait été découverte pour une première utilisation, ce qui oblige à limiter la durée de vie des flacons en cours d'utilisation dans le souci de prévenir l'apparition d'infections oculaires parasites.

Certains ont encore pensé pouvoir traiter le problème en adoptant des dispositions qui existent dans d'autres secteurs, où l'on réalise des circuits de circulation d'eau qui sont constitués en un matériau présentant une activité bactéricide. C'est le cas du document de brevet WO 2007/056131, qui évite le recours à une membrane filtrante anti-bactérienne en prévoyant de traiter ainsi une valve anti-retour au travers de laquelle le liquide est expulsé à partir d'un flacon. Au fait que cette solution n'a pas fait preuve de l'efficacité recherchée, il s'ajoute qu'elle est réservée aux flacons à déformation irréversible, dans lesquels le réservoir de liquide voit son volume diminuer à chaque utilisation, aucune rentrée d'air ne venant en remplacement du liquide consommé.

Or la présente invention vise essentiellement à améliorer les conditions sanitaires à l'utilisation d'un flacon de conditionnement de liquide dans lequel le réservoir de liquide est à paroi à déformation élastiquement réversible par admission d'air à l'intérieur du réservoir en remplacement de tout volume de liquide expulsé et dans lequel l'admission d'air s'effectue à travers la tête de distribution par la même voie que l'expulsion de liquide. Elle vise aussi à profiter pleinement des possibilités qu'offrent les membranes anti-bactériennes en proposant un flacon convenant au conditionnement de solutions ophtalmiques dépourvues de conservateur.

A l'origine de l'invention, on a observé qu'avec ce type de flacons, le risque d'une contamination oculaire ne vient pas tant du liquide instillé que du flaconnage lui-même, dont l'embout est exposé à être touché par l'utilisateur, à rester privé de son capuchon entre deux utilisations et à s'approcher de trop près des paupières.

Dans le flacon proposé par l'invention, voir aussi revendication 1, la tête de distribution de liquide goutte à goutte comportant une membrane filtrante anti-bactérienne qui est interposée sur le trajet du liquide expulsé et de l'air rentrant en compensation et qui est montée entre une nacelle de montage étanche sur le flacon en communication avec son réservoir interne et l'embout compte-gouttes, il est prévu de réaliser l'embout seul, à l'exclusion de la nacelle, en un matériau contenant un agent bactéricide ayant effet en prévention anti-bactérienne d'une prolifération bactérienne sur sa surface extérieure.

Des flacons comprenant une telle membrane filtrante anti-bactérienne sont déjà connus en soi, ils évitent que les bactéries que pourrait porter l'air entrant dans le flacon après l'expulsion d'une goutte de liquide ne pénètrent jusque dans le réservoir pour contaminer le liquide restant. Cette membrane ne comporte pas d'agent bactéricide, son action anti-bactérienne provient de ses propriétés filtrantes : elle empêche les bactéries de la traverser, le diamètre moyen de pore de la membrane étant inférieur ou égal à 0,2 µm (par exemple de 0,1 à 0,2 µm). La présence de cette membrane permet d'utiliser le flacon pour conditionner un liquide qui n'a pas besoin de contenir un agent conservateur, à condition toutefois de n'utiliser le produit que pendant un temps limité après ouverture du flacon. On préconise d'une manière générale un mois d'utilisation tout au plus.

Plus spécifiquement encore l'invention vise un flacon de conditionnement d'un liquide qui comporte un réservoir à paroi à déformation élastiquement réversible par admission d'air à l'intérieur du récipient, pour délivrer le liquide sous l'effet d'une pression exercée contre cette paroi et pour autoriser un retour spontané du récipient à sa conformation initiale après délivrance d'une dose de liquide, la membrane filtrante anti-bactérienne étant partiellement hydrophile et partiellement hydrophobe. Le principe de fonctionnement d'un tel flacon a été décrit en détails dans la demande de brevet internationale WO2006/000897.

Dans le cas de tels flacons, on utilise une membrane bifonctionnelle, partiellement hydrophile et partiellement hydrophobe, par exemple en polymère à base de polyamide ou de polyéther sulfone, qui autorise d'une part le passage du liquide dans le sens de la distribution, sous l'effet d'une pression exercée par l'utilisateur sur la paroi du réservoir, et d'autre part le passage de l'air en sens inverse, de l'extérieur vers le réservoir, lorsque cette pression est relâchée après la distribution d'une goutte de liquide. On sait se procurer dans le commerce une membrane filtrante, qui est rendue partiellement hydrophobe sur une partie de sa surface, par modification de sa structure notamment par greffage en présence d'un initiateur de réactions radicalaires, de manière à autoriser le passage de l'air de l'extérieur vers le réservoir après chaque opération de distribution. Ce traitement est notamment réalisé sur une bande médiane occupant 20 à 50 % de sa surface disposée au travers du chemin du liquide.

Par ailleurs, un flacon tel que décrit dans la demande de brevet internationale WO2006/000897 comporte un tampon microporeux hydrophobe régulateur du flux de liquide à délivrer, situé entre le réservoir de stockage du liquide et la membrane filtrante. Il comporte aussi un capuchon de protection de l'embout créant une étanchéité quand le flacon n'est pas utilisé pour délivrer du liquide.

Selon l'invention, seul l'embout compte-gouttes, qui est situé au-delà de cette membrane filtrante anti-bactérienne, est traité avec un agent bactéricide. De ce fait, le liquide restant dans le réservoir ne vient pas en contact avec les surfaces des parties où le matériau contient un agent bactéricide. Ceci évite ainsi que les agents bactéricides altèrent au cours du temps le liquide restant dans le réservoir. A supposer même que des fractions minimes de liquide puissent rentrer jusque dans le flacon après expulsion goutte-à-goutte du liquide, ce liquide tout comme l'air aspiré en équilibrage de pression aura été filtré de toute bactérie au passage de la membrane avant de pénétrer dans le réservoir.

Dans la demande de brevet WO 2007/056131 on a proposé un flacon de conditionnement d'un liquide à distribuer goutte à goutte avec un embout formant une valve par laquelle le liquide est expulsé sans permettre la moindre admission d'air dans le réservoir contenant le liquide. Quand dans ce document, il est préconisé de plus que les matériaux constituant cet embout ou tout l'ensemble du flacon soient traités par un agent bactéricide, il ne peut s'agir que de traiter le liquide à son passage.

Selon un mode de mise en oeuvre préféré de l'invention, le corps de l'embout compte-gouttes est formé en une matière, notamment en une matière plastique moulée, qui contient un polymère porteur d'ions à effet bactéricide uniformément réparti dans sa masse.

Le polymère porteur d'ions choisi dans le cadre de l'invention présente avantageusement, grâce aux propriétés bactéricides des ions qu'il porte, une action anti-microbienne efficace contre les souches bactériennes mais également contre les levures et les moisissures. Les ions en questions sont en particulier des ions argent. Il a été constaté par les inventeurs que les flacons équipés d'un tel embout compte-gouttes assurent avantageusement une bonne sécurité biologique du patient, tout en ne présentant pas de toxicité pour l'oeil.

L'effet anti-bactérien s'exerce avantageusement sur la surface de l'embout compte-gouttes. Les ions bactéricides qui se trouvent présents sur cette surface, et notamment sur la surface qui risque d'entrer en contact avec l'oeil, exercent à cet endroit un effet bactériostatique, inhibant la prolifération bactérienne qui peut débuter après la contamination de l'embout. Cette contamination bactérienne peut par exemple provenir des bactéries présentes dans l'oeil ou autour de celui-ci, par un contact de l'embout avec les tissus ou les fluides oculaires lors de l'administration des gouttes. Cette contamination peut également émaner, plus généralement, du contact volontaire ou non avec les doigts de l'utilisateur au moment de la distribution, ou tout simplement de l'air ambiant ou d'un corps étranger lorsque l'embout n'est pas protégé par un capuchon entre deux utilisations. De plus, des résidus de liquide, de l'ordre de quelques microlitres, sont toujours ré-aspirés dans l'embout après chaque opération de distribution, ne serait-ce que dans le canal d'expulsion. Ces résidus constituent un milieu humide favorable susceptible de former une source pour une prolifération microbienne dans l'embout.

Au fil du temps et des différentes utilisations, on peut penser que les ions bactéricides présents dans la matière migrent au sein du polymère qui les porte, en direction de la surface de l'embout, de manière à remplacer les ions consommés au fur et à mesure de leur action à partir de cette surface.

L'embout compte-gouttes selon l'invention reste ainsi avantageusement toujours protégé, sur sa surface tant externe qu'interne, de la prolifération microbienne qui serait susceptible d'engendrer des concentrations bactériennes suffisamment importantes pour être nocives pour la santé ou le confort de l'utilisateur. En particulier, on évite ainsi que soit contaminé le liquide qui traverse l'embout au moment de la distribution. L'embout ne constitue en outre jamais une source de contamination directe de l'oeil, par contact de l'embout avec l'oeil au moment d'une distribution ultérieure. Dans le cas des embouts de distribution intégrant à leur base une membrane anti-bactérienne pour empêcher la contamination du liquide à l'intérieur du réservoir, on diminue également le risque qu'il se forme, sur la surface de l'embout compte-gouttes, un film biologique, ou biofilm, qui contaminerait systématiquement le liquide au moment de son passage à travers la membrane au cours de la distribution.

Le procédé de fabrication de l'embout selon l'invention est avantageusement tout à fait simple à mettre en oeuvre. Les particules de polymère chargé d'ions à effet bactéricide sont introduites dans la matière plastique, de manière à obtenir après chauffage un mélange homogène, à partir duquel est réalisé le processus de moulage de l'embout.

Ce procédé ne requiert, par rapport aux procédés classiques de fabrication des embouts de distribution par moulage, qu'une seule et simple étape supplémentaire, c'est-à-dire le mélange du polymère anti-bactérien avec la matière principale formant l'embout à mouler en tout début du procédé de moulage.

Les ions argent sont tout particulièrement préférés en tant qu'ions à effet bactéricide. Ces ions sont connus de façon classique pour leurs propriétés anti-microbiennes, alors qu'ils ne présentent pas de toxicité pour l'oeil humain dans la concentration de quelques pourcents de polymère porteur des ions argent dans la masse. Ils sont efficaces contre la plupart des bactéries, levures, champignons et autres microbes similaires. Ils se lient à la membrane des cellules et perturbent sa fonction naturelle. Ils pénètrent également à travers les parois des cellules, dans lesquelles ils se combinent avec des groupements donneurs d'électrons et des groupements chargés négativement, ainsi qu'avec des groupes thiol fréquents dans les enzymes. Ceci induit un dysfonctionnement des cellules qui entraîne rapidement leur mort.

Les ions argent sont par exemple introduits dans la matière sous forme de granules les contenant en fine dispersion dans du polyéthylène, portés par une résine échangeuse d'ions inorganique. Il existe dans le commerce de telles granules, qui présentent notamment l'avantage d'une grande facilité et sécurité d'utilisation.

La proportion de particules de polymère porteur d'ions bactéricides qui sont incorporées dans le mélange pour la formation du corps de l'embout est avantageusement comprise entre 1 et 10 %, et de préférence entre 2 et 5 %, en poids du poids total du mélange.

L'embout compte-gouttes selon l'invention peut être formé en une seule pièce annulaire, percée d'un canal central étroit pour l'expulsion du liquide.

Selon un mode de réalisation particulièrement avantageux, de l'invention l'embout compte-gouttes est réalisé de manière à répartir la section de passage offerte à la circulation du liquide expulsé ou de l'air ré-aspiré en plusieurs circuits limités par des surfaces de matériau chargé d'agent bactéricide. A cet effet, l'invention prévoit notamment de ménager dans l'embout un canal central interne relativement large dans lequel est inséré un noyau central, formant une pluralité de sous-canaux pour l'expulsion du liquide, et de réaliser ce noyau central en un matériau contenant un agent bactéricide réparti dans sa masse.

Un avantage de ce noyau central est que l'efficacité en terme d'inhibition de la prolifération bactérienne de l'embout est encore améliorée, puisque la surface de contact offerte à l'air pénétrant de l'extérieur, et donc à la source de contamination bactérienne extérieure, par les parois en matière anti-microbienne des sous-canaux, est plus importante que dans le cas d'un canal unique.

Selon une caractéristique avantageuse de l'invention, le noyau central comporte un agent bactéricide différent de celui contenu dans le corps externe de l'embout (la partie extérieure). Ceci permet d'étendre l'efficacité contre la prolifération bactérienne, on peut en effet combiner des agents bactéricides ayant des spectres anti-bactériens différents et les mieux adaptés selon les parties de l'embout. Comme explicité plus haut, les agents bactéricides porteurs d'ions comme les ions argent libèrent ces ions bactéricides au cours du temps et ont une action intéressante pour les surfaces externes de l'embout et pour lutter contre la formation de biofilms.

Selon une caractéristique particulière de l'invention, le noyau central comprend comme agent bactéricide un composé choisi parmi les composés phénoliques, et notamment les composés phénoliques chlorés. De préférence ce composé phénolique chloré est le 5-chloro-2-(2,4-dichlorophénoxy)phénol, connu sous le nom de triclosan. Ce composé présente un large spectre anti-bactérien. Ce composé a selon certaines études une action biocide par action sur la membrane et/ou le cytoplasme des bactéries et une action bactériostatique empêchant la prolifération des bactéries en inhibant principalement la synthèse d'acides gras nécessaire à la reproduction et construction des membranes cellulaires.

Selon une caractéristique avantageuse de l'invention, le noyau central est formé en la même matière de base que celle utilisée pour former le corps principal externe de l'embout compte-gouttes.

Le noyau central et le corps principal externe de l'embout, qui délimite le canal central, sont fabriqués chacun indépendamment de l'autre dans des opérations de moulage distinctes, puis assemblés l'un à l'autre, par insertion du noyau dans le canal.

Dans un tel mode de réalisation, l'invention prévoit avantageusement que les sous-canaux soient formés par des rainures creusées sur la surface externe du noyau. Cette caractéristique s'avère particulièrement avantageuse car elle permet une meilleure maîtrise de l'état de surface des parois du canal d'expulsion, en liaison avec le procédé de fabrication de l'embout.

En effet, dans le cas des embouts classiques, c'est-à-dire comportant un canal central étroit pour l'expulsion du liquide, la formation du canal est réalisée après le procédé de moulage, par perçage au moyen d'une aiguille très fine dans la matière encore incomplètement durcie. Au niveau industriel, un tel procédé peut créer des micro irrégularités sur la surface du canal central, engendrant la formation d'éventuelles micro-poches de rétention sur la paroi épaisse du canal. Ces micro-poches constituent des niches pour la prolifération des bactéries.

Dans l'embout selon l'invention, le canal d'expulsion du liquide comprend une pluralité de sous-canaux. Ces sous-canaux, de par leur procédé de fabrication, selon lequel les rainures sont formées lors du moulage du noyau au moyen d'un moule de forme adéquate, présentent des parois plus lisses, qui sont dépourvues d'aspérités de surface dans lesquelles des bactéries et autres microbes pourraient venir se loger et proliférer.

Les sous-canaux sont de préférence au moins au nombre de deux, et de préférence au nombre de quatre, régulièrement répartis autour de l'axe du noyau, de manière à assurer une surface de contact importante avec l'air aspiré dans l'embout après chaque opération de distribution.

Selon un autre mode préféré de l'invention, le noyau central est un matériau thermoplastique poreux, en particulier à base de polyoléfines, et plus particulièrement choisis parmi la familles des polyéthylènes (comme peut l'être le corps principal externe de l'embout). Les polyéthylènes confèrent à ce matériau un caractère hydrophobe qui évite les stagnations de liquide.

Un tel matériau poreux est de préférence un matériau fritté. Il est obtenu par frittage, c'est-à-dire par traitement thermique de particules du polymère thermoplastique, préalablement comprimées à froid dans un moule, réalisé à une température inférieure à celle du point de fusion du polymère (constituant principal). Ce procédé de fabrication permet de lier les particules entre elles sans les faire fondre et de contrôler la porosité du matériau en agissant notamment sur la température et la pression. L'agent bactéricide peut être ajouté de diverses manières, en masse avec le polymère de base, par malaxage d'un polymère traité bactéricide et du polymère de base, ou encore par des traitements de surface avec ou sans additifs. Selon un cas particulier, le matériau polymère poreux est réalisé par frittage puis le fritté obtenu est traité par l'agent bactéricide. De tels matériaux poreux et leurs procédés de fabrication sont décrits par exemple dans la demande de brevet internationale WO01/65937.

Avantageusement, dans ce mode de réalisation de l'invention, le matériau thermoplastique poreux a une dimension moyenne de pore dont l'ordre de grandeur est de la centaine de micromètres. Cette dimension peut par exemple être comprise entre 0,1 et 0,2 mm (100 et 200 µm).

Avantageusement aussi dans ce mode de réalisation de l'invention, le matériau poreux et la partie terminale de la partie extérieure de l'embout ont des formes cylindriques similaires, en concordance c'est-à-dire qui s'épousent. Ceci permet d'éviter que stagne du liquide entre la paroi de cette partie de l'embout et le noyau central. Cette forme cylindrique du noyau central est un compromis qui minimise les pertes de charge lorsque le liquide le traverse pour être distribué et par ailleurs optimise les surfaces de contact anti-bactériennes. Selon un cas particulier de ce mode de réalisation, la partie terminale de l'embout a une forme surmoulée au matériau poreux. Elle est faite par surmoulage du matériau poreux afin que leurs formes s'épousent exactement.

Dans des modes de réalisation préférés de l'invention, l'embout comporte, à la base du canal central, un bosselage périphérique qui coopère avec une rainure périphérique complémentaire formée à la base du noyau, pour la fixation par effet d'encliquetage élastique du noyau dans le canal. Le noyau est inséré en force dans le canal, et il est fermement maintenu à l'intérieur de celui-ci par le bosselage formé à sa base. Le bosselage et la rainure sont formés lors de l'opération de moulage de l'embout, respectivement de sa partie extérieure, délimitant le canal central, et de son noyau.

L'invention sera maintenant plus complètement décrite dans le cadre de caractéristiques préférées et de leurs avantages, en faisant référence aux figures 1 à 4 dans lesquelles :
- la figure 1 représente une vue en coupe selon un plan longitudinal montrant l'embout d'une tête de distribution suivant l'invention ;
- la figure 2 illustre en vue en perspective le noyau central de l'embout de la figure 1, et en vue en coupe selon un plan longitudinal son logement de réception dans l'embout ;
- la figure 3 montre une vue en coupe selon le plan A-A de l'embout de la figure 1 ;
- et la figure 4 représente une vue en coupe selon un plan longitudinal d'un embout avec un noyau central poreux selon l'invention.

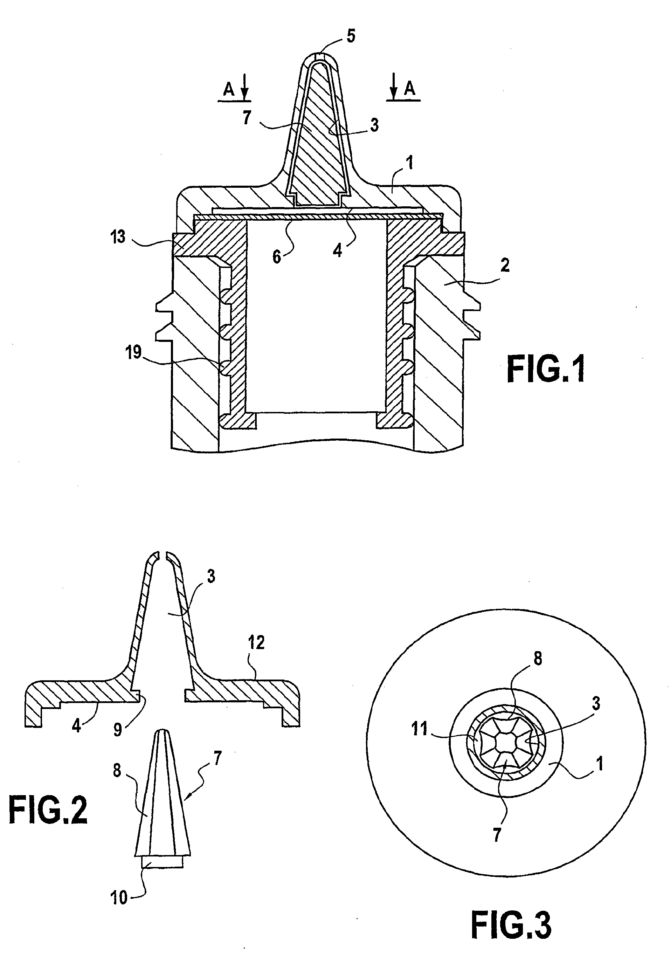

Un exemple d'un premier type d'embout de distribution 1 d'une tête de distribution selon l'invention est représenté sur la figure 1.

La tête de distribution comprend une nacelle 13, qui est prévue pour être montée de façon étanche, grâce à des godrons d'étanchéité 19, à l'intérieur du col du flacon 2. L'embout 1 la prolonge suivant son axe. Il lui est soudé par son embase 4.

Le flacon comprend un réservoir, non représenté sur la figure, limité par une paroi à déformation élastique réversible, qui est destiné à contenir un collyre, avantageusement dépourvu de conservateur. Quand on presse manuellement sur la paroi souple du réservoir, le liquide est forcé à travers un tampon régulateur de flux disposé dans le conduit interne de la nacelle 13. Le retour spontané du réservoir à sa forme d'origine provoque une entrée d'air par le même conduit.

L'embout comporte un canal central longitudinal 3, qui le traverse sur toute sa hauteur depuis la base 4 de l'embout jusqu'à l'orifice 5 d'expulsion du liquide, situé à son extrémité supérieure (en considérant le flacon posé à la verticale).

Sous la base de l'embout 1, une membrane filtrante anti-bactérienne 6 est disposée en travers du passage du liquide du réservoir à l'embout et de l'air entrant. Cette membrane est destinée à protéger le liquide contenu dans le réservoir des contaminations extérieures.

Le corps 12 de l'embout est constitué en une matière plastique, notamment en polymère du type polyéthylène, incorporant dans la masse un polymère porteur d'ions à effet bactéricide. Ce dernier est choisi pour être compatible avec la matière classique de l'embout. Ne serait-ce que pour cette raison, il est de préférence à base de polyéthylène. Il est disponible dans le commerce sous forme de poudre ou de granules ou billes, prêt à être incorporé dans la composition de moulage de l'embout. L'agent bactéricide est de préférence constitué d'ions argent, qui sont portés par les macromolécules de polymère.

De tels ions argent sont connus pour être efficaces sur de nombreuses souches bactériennes, de levures et de moisissures, notamment sur les souches *pseudomonas* et *staphylococcus,* les plus couramment présentes sur la peau et les muqueuses oculaires.

A titre de produits commerciaux constitués de polymère, notamment de polyéthylène, chargé d'ions argent, pouvant être utilisés dans le cadre de l'invention, on peut par exemple citer I'AlphaSan® de la société Clariant S.p.A., ou le Biomaster commercialisé par la société Addmaster Ltd.

L'embout selon l'invention est fabriqué selon un procédé de moulage classique à partir du mélange contenant le polymère anti-microbien en mélange homogène avec le polyéthylène. La proportion de granules de polymère porteur d'ions argent dans le polyéthylène est d'environ 5 % en poids.

A l'issue du procédé de moulage, l'agent bactéricide est présent dans toute la masse de l'embout, et en particulier tant sur sa surface externe susceptible d'entrer en contact avec les yeux ou les mains de l'utilisateur, que sur sa surface interne délimitant son canal central 3.

A l'intérieur du canal central 3 de l'embout est disposé un noyau central 7, qui est représenté en vue en perspective sur la figure 2. Le noyau 7 présente une forme complémentaire de celle du canal central 3 dans lequel il est logé, c'est-à-dire une forme globalement conique s'évasant du haut vers le bas. Son diamètre externe est ajusté au diamètre interne du canal 3, de telle sorte que le liquide ne peut circuler entre le canal et le noyau.

Sur la surface externe du noyau 7 sont formées quatre rainures 8, régulièrement réparties autour de l'axe du noyau 7.

Le noyau central 7 est fabriqué par un procédé de moulage, à partir du même matériau de base, notamment en polyéthylène, que le corps de l'embout 12 qui l'entoure, mais il comprend avantageusement un agent bactéricide différent de celui contenu dans le corps 12 pour avoir effet à la surface extérieure de l'embout. Cet agent bactéricide est ici le triclosan. Le triclosan a un large spectre antibactérien (et aussi anti-fongique). Les rainures 8 à sa surface sont formées lors du procédé de moulage, par une forme spécifiquement adaptée du moule.

Lorsque le noyau 7 est disposé dans le canal 3, comme illustré sur la figure 3, il se forme entre la paroi externe du noyau 7 et la paroi interne du canal 3, au niveau des rainures 8, des sous-canaux 11 de faible section. Ces sous-canaux 11 assurent le passage du liquide de la base de l'embout jusqu'à l'orifice d'expulsion 5. Leur surface de contact avec l'air est lisse et régulière, sans zone de stagnation pour l'air, le liquide et les bactéries.

A la base du canal central 3, l'embout 1 comporte un bosselage annulaire 9 réduisant le diamètre du canal à cet endroit. Le noyau 7 comporte quant à lui, également à sa base, une rainure annulaire 10 complémentaire du bosselage 9.

- La partie extérieure 12 de l'embout 1 et le noyau 7 sont fabriqués chacun par un procédé de moulage classique, puis assemblés l'un à l'autre.

Lors du montage de l'embout selon l'invention, le noyau 7 est inséré par engagement en force dans le canal 3, jusqu'à buter contre l'extrémité supérieure de l'embout entourant le canal d'expulsion, qui forme la couronne de libération de la goutte. Dans cette position, la rainure 10 se trouve en vis-à-vis du bosselage 9. Ces deux éléments coopèrent par un effet d'encliquetage élastique, pour assurer un maintien solide du noyau à l'intérieur du canal.

Suivant son mode de mise en oeuvre préféré, l'invention prévoit de remplacer le noyau à rainures précédent par un noyau poreux, inséré de la même manière dans le canal d'expulsion traversant l'embout, pour assurer la même fonction de diviser le flux de liquide en le répartissant dans une pluralité de circuits à travers le canal d'expulsion percé dans l'embout.

Les avantages en sont multiples. D'une part, le flux est beaucoup plus divisé et la répartition est plus fine. D'autre part, le canal peut être mieux rempli de manière uniforme par la matière léchée par le liquide lors de son expulsion. D'autre part encore, c'est en combinaison avec la forme poreuse que le recours à un agent bactéricide de la famille des composés organiques phénoliques chlorés plutôt que d'un agent ionique présente le plus d'intérêt pour l'efficacité contre les risques de contamination oculaire.

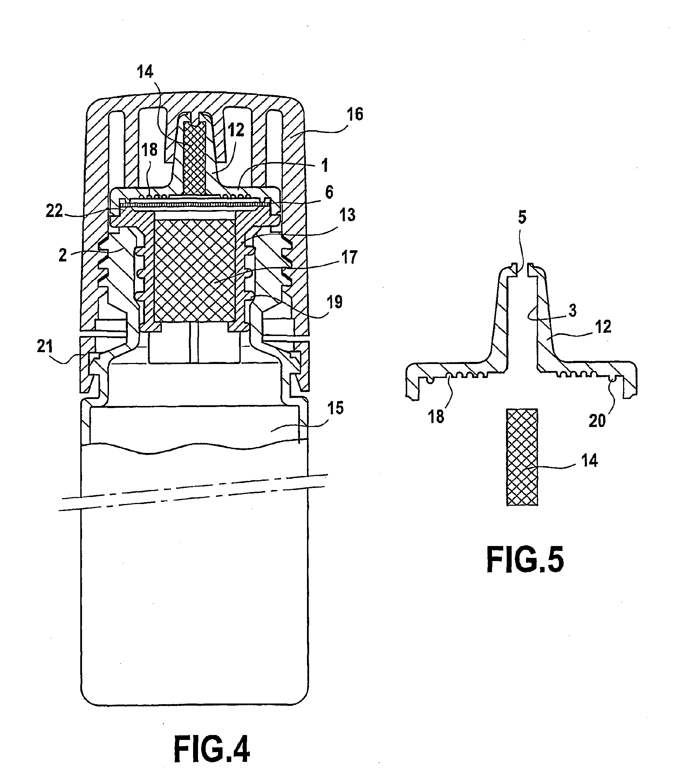

Un flacon équipé d'une telle tête de distribution est représenté sur la figure 4.

Comme précédemment, la tête de distribution comprend une nacelle 13 à laquelle est solidarisé l'embout 1 pour former un insert qui a été introduit en force dans le col 2 du flacon. A l'opposé de l'embout, la nacelle se termine dans le flacon par quatre parois radiales en croix.

La nacelle 13 de la tête de distribution est montée de façon étanche, grâce à des godrons d'étanchéité 19, à l'intérieur du col du flacon 2, d'où dépasse l'embout 1 qui se prolonge dans l'axe du flacon. L'intérieur du flacon ménage un réservoir 15, limité par une paroi cylindrique à déformation élastique réversible, qui est destiné à contenir un collyre, avantageusement dépourvu de conservateur. Un capuchon de protection 16 et une bague d'inviolabilité 21 complètent l'ensemble extérieurement.

Le col du flacon 2 comporte, sous l'embase de l'embout 1, une membrane filtrante anti-bactérienne 6, disposée en travers du passage du liquide du réservoir à l'embout et de l'air entrant. Elle est librement supportée en fonctionnement par application contre l'embase de l'embout. Elle est fixée sur son pourtour par soudage thermique entre une couronne périphérique de cette embase (qui présente là un bourrelet qui s'estompe lors de l'opération de soudage entre les deux pièces) et une portée coopérante 22 en face terminale de la nacelle.

Un tampon microporeux 17 est disposé dans l'alésage central de la tête de distribution. Il est en lui-même classique, y compris dans sa fonction en régulation des flux de liquide et équilibrage des pressions d'air. Sa structure est celle d'un feutre de fils entremêlés, sous une densité correspondant à un diamètre de pores équivalent de l'ordre de 50 microns.

Des gorges circulaires 18 permettent de drainer le flux du liquide forcé à travers la membrane filtrante 6 vers le canal d'expulsion central 3.

Le corps 12 de l'embout est fait comme décrit précédemment pour l'autre embout de la figure 1 mais sa partie supérieure est cylindrique (au lieu d'être conique). Ce corps principal 12 est fait d'une matière polymère, à base de polyéthylène, comportant un agent bactéricide polymère porteur d'ions argent.

Le noyau central 14, qui est représenté en vue en perspective sur la figure 5, est un noyau cylindrique poreux, de forme complémentaire à celle de la partie supérieure de l'embout, et d'une taille remplissant le canal central 3 de l'embout. Ce noyau poreux occupe tout l'espace du canal central 3 pour avoir la plus grande surface possible de contact avec les micro-gouttes de liquide ou l'air rentrant dans l'embout après expulsion d'une goutte de liquide lors de son utilisation. Ceci évite aussi les pertes de charge engendrées lorsqu'il est traversé par le liquide sortant.

Ce noyau poreux a une longueur dans l'ordre de grandeur du centimètre, soit notamment une longueur comprise entre 0,5 et 2,5 cm, et un diamètre dans l'ordre de grandeur du millimètre, plus particulièrement compris entre 2 et 6 millimètres.

Ce noyau central 14 est fait d'un matériau thermoplastique poreux, à base de polyéthylène fritté, comportant un agent bactéricide, constitué d'une molécule organique diffusée dans la masse du polymère, et non plus d'un polymère à ions métalliques comme pour le corps de l'embout. Dans cet exemple, le noyau en matériau fritté 14 comporte du triclosan alors que la partie extérieure 12 de l'embout comporte un agent bactéricide à base d'ions argent.

Son diamètre de pore moyen est dans l'ordre de grandeur du dixième de millimètre, et plus particulièrement de l'ordre de 150 µm. Un tel matériau poreux est notamment commercialisé par la société POREX Corporation.

Ce noyau central 14 a été introduit en force dans le canal central 3 de l'embout.

Selon un autre mode de mise en oeuvre, la partie extérieure 12 de l'embout est faite par surmoulage du noyau poreux 14. Ce surmoulage permet d'avoir un embout dont les formes respectivement du noyau central 14 et de la partie du canal central 3 sont en parfaite concordance. Ceci permet d'éviter que stagne du liquide entre la paroi de cette partie de l'embout et le noyau central.

Les têtes de distribution des flacons avec les deux types d'embouts présentés en exemples selon l'invention ainsi constitués présentent un effet anti-microbien important, tant au niveau de la paroi externe de l'embout, susceptible d'être contaminée par l'environnement extérieur, que des parois des canaux d'expulsion du liquide qui sont susceptibles d'être contaminées par l'air et les résidus de liquide ré-aspirés depuis l'extérieur après chaque opération de distribution de liquide. Cet effet anti-microbien, qui s'est avéré efficace contre les contaminants courants en milieu oculaire, inhibe la prolifération bactérienne sur ces surfaces, et permet de conserver un embout sain, assurant la sécurité microbiologique de l'oeil du consommateur.

La description qui précède explique clairement comment l'invention permet d'atteindre les objectifs qu'elle s'est fixés. En particulier, elle fournit un flacon de conditionnement de liquide, notamment ophtalmique, avec un embout compte-gouttes qui conserve par un effet anti-bactérien, un état microbiologiquement sain pendant toute sa durée d'utilisation, et qui assure de ce fait une bonne sécurité microbiologique pour l'utilisateur.

Il ressort néanmoins de ce qui précède que l'invention n'est pas limitée aux modes de mise en oeuvre qui ont été spécifiquement décrits et représentés sur les figures et qu'elle s'étend au contraire à toute variante passant par le biais de moyens équivalents.

## Revendications

1. Flacon de conditionnement d'un liquide à distribuer goutte à goutte comprenant un réservoir de réception d'un liquide sans agent conservateur, ledit réservoir étant à paroi à déformation élastiquement réversible par admission d'air à l'intérieur du réservoir par l'intermédiaire d'une tête de distribution par laquelle le liquide est délivré sous l'effet d'une pression exercée contre ladite paroi,
- dans lequel ladite tête de distribution du liquide comporte une nacelle (13), qui se monte de manière étanche dans le flacon, ainsi qu'un embout compte-gouttes (1) qui la prolonge à l'extérieur du flacon et qui est percé d'un canal central (3) débouchant par un orifice d'expulsion du liquide,
- et dans lequel une membrane filtrante anti-bactérienne (6), montée à la base (4) dudit embout, entre celui-ci et ladite nacelle, est interposée en travers de la circulation de l'air appelé à rentrer dans le flacon après une distribution de liquide ;
**caractérisé en ce que** sélectivement ledit embout compte-gouttes (1) situé au-delà de ladite membrane filtrante anti-bactérienne, à l'exclusion de ladite nacelle (13), est réalisé en un matériau contenant un agent bactéricide ayant effet en prévention d'une prolifération bactérienne à la surface du matériau,
et **caractérisé en ce que** l'embout (1) est réalisé en deux pièces comprenant un corps (12) et un noyau central (7), ledit noyau central étant inséré dans le canal central de l'embout et comportant une pluralité de sous canaux de manière à définir le circuit d'expulsion de liquide à travers ledit corps, ledit corps et ledit noyau central étant réalisés tous les deux en des matériaux contenant des agents bactéricides.

2. Flacon selon la revendication 1, **caractérisé en ce qu'**il comporte en outre un tampon régulateur de flux dans ladite nacelle (13).

3. Flacon selon la revendication 1 ou 2, **caractérisé par le fait que** le matériau dudit corps (12) contient un agent bactéricide réparti dans la masse ayant effet par migration vers la surface du matériau tandis que ledit noyau central (7) est réalisé en un matériau comportant un agent bactéricide à base d'un composé phénolique chloré.

4. Flacon suivant la revendication 3, **caractérisé par le fait que** ledit composé phénolique chloré que comporte ledit noyau (7) est le 5-chloro-2-(2,4-dichlorophénoxy)phénol, ou triclosan.

5. Flacon suivant l'une des revendications précédentes, **caractérisé par le fait que** ledit corps (12) de l'embout est constitué en une matière contenant comme agent bactéricide un polymère porteur d'ions qui est uniformément réparti dans sa masse.

6. Flacon selon la revendication 5, **caractérisé en ce que** lesdits ions bactéricides sont des ions argent.

7. Flacon selon l'une des revendications précédentes, **caractérisé en ce que** le noyau (7) interne à l'embout (1) est un matériau thermoplastique fritté poreux, en particulier à base de polyéthylène.

8. Flacon selon la revendication 7, **caractérisé en ce que** le matériau poreux présente une dimension moyenne de pores dont l'ordre de grandeur est de la centaine de micromètres.

9. Flacon selon l'une des revendications 7 ou 8, **caractérisé par le fait que** la partie terminale du canal d'expulsion du corps principal externe de l'embout et le noyau en matériau poreux ont une forme cylindrique, la forme cylindrique de la partie terminale du canal d'expulsion épousant la forme cylindrique du noyau en matériau poreux.

10. Flacon selon la revendication 9, **caractérisé par le fait que** le corps (12) de l'embout est réalisé par surmoulage sur le matériau poreux constitutif dudit noyau.

11. Flacon selon l'une quelconque des revendications précédentes, **caractérisé en ce qu'**il est utilisé pour le conditionnement d'une solution ophtalmique dépourvue de conservateur.

## Patentansprüche

1. Fläschchen zum Konditionieren einer tropfenweise abzugebenden Flüssigkeit, die ein Reservoir zur Aufnahme einer Flüssigkeit ohne Konservierungsmittel umfasst, wobei das Reservoir eine Wand aufweist, die durch Lufteinlass in das Reservoir über einen Verteilerkopf, durch den die Flüssigkeit unter der Wirkung eines auf die Wand ausgeübten Drucks abgegeben wird, elastisch reversibel verformbar ist,
- wobei der Verteilerkopf zur Abgabe der Flüssigkeit einen Einsatz (13) aufweist, der abgedichtet in dem Fläschchen angebracht wird, sowie einen Aufsatz (1) zur tropfenweisen Flüssigkeitsabgabe, durch den der Einsatz außerhalb dem Fläschchen verlängert wird und durch den ein Mittenkanal (3) führt, welcher in einer Öffnung zum Ausstoßen der Flüssigkeit mündet,
- und wobei eine antibakterielle Filtermembran (6), die an der Grundseite (4) des Aufsatzes zwischen diesem und dem Einsatz angebracht ist, quer zur Strömung der Luft, die nach der Flüssigkeitsabgabe in das Fläschchen zurückströmen soll, eingesetzt ist;
**dadurch gekennzeichnet, dass** der jenseits der antibakteriellen Filtermembran angeordnete Aufsatz (1) zur tropfenweisen Flüssigkeitsabgabe unter Ausschluss des Einsatzes (13) selektiv aus einem Material ausgeführt ist, das einen bakteriziden Wirkstoff enthält, der eine vorbeugende Wirkung gegen die Ausbreitung von Bakterien an der Oberfläche des Materials hat,
und **dadurch gekennzeichnet, dass** der Aufsatz (1) zweiteilig ausgeführt ist und einen Körper (12) und einen Zentralkern (7) umfasst, wobei der Zentralkern in den Mittenkanal des Aufsatzes eingesetzt wird und eine Vielzahl von Teilkanälen aufweist, so dass der Weg zum Ausstoßen der Flüssigkeit durch den Körper vorgegeben wird, wobei sowohl der Körper als auch der Zentralkern aus Materialien ausgeführt sind, die bakterizide Wirkstoffe enthalten.

2. Fläschchen nach Anspruch 1, **dadurch gekennzeichnet, dass** sie des Weiteren einen den Durchfluss regelnden Stopfen in dem Einsatz (13) aufweist.

3. Fläschchen nach Anspruch 1 oder 2, **dadurch gekennzeichnet, dass** das Material des Körpers (12) einen bakteriziden Wirkstoff enthält, der in der Masse verteilt ist und durch Migration zur Oberfläche des Materials hin wirkt, während der Zentralkern (7) aus einem Material ausgeführt ist, das eine bakteriziden Wirkstoff auf der Basis einer chlorierten Phenolverbindung aufweist.

4. Fläschchen nach Anspruch 3, **dadurch gekennzeichnet, dass** es sich bei der chlorierten Phenolverbindung, die der Kern (7) aufweist, um 5-Chlor-2-(2,4-dichlorophenoxy)phenol oder Triclosan handelt.

5. Fläschchen nach einem der vorhergehenden Ansprüche, **dadurch gekennzeichnet, dass** der Körper (12) des Aufsatzes aus einem Material besteht, das als bakteriziden Wirkstoff ein Ionen tragendes Polymer enthält, das gleichmäßig in seiner Masse verteilt ist.

6. Fläschchen nach Anspruch 5, **dadurch gekennzeichnet, dass** es sich bei den bakteriziden Ionen um Silber-Ionen handelt.

7. Fläschchen nach einem der vorhergehenden Ansprüche, **dadurch gekennzeichnet, dass** der Kern (7) innerhalb des Aufsatzes (1) ein poröses gesintertes thermoplastisches Material ist, insbesondere auf der Basis von Polyethylen.

8. Fläschchen nach Anspruch 7, **dadurch gekennzeichnet, dass** das poröse Material eine mittlere Porengröße in der Größenordnung von Hundertstel Mikrometern aufweist.

9. Fläschchen nach einem der Ansprüche 7 oder 8, **dadurch gekennzeichnet, dass** das Endstück des Ausstoßkanals des Hauptkörpers außerhalb des Aufsatzes und der Kern aus porösem Material eine zylindrische Form haben, wobei die zylindrische Form des Endstücks des Ausstoßkanals und die zylindrische Form des Kerns aus porösem Material passgerecht zueinander sind.

10. Fläschchen nach Anspruch 9, **dadurch gekennzeichnet, dass** der Körper (12) des Aufsatzes durch Umspritzen des porösen Materials, aus dem der Kerns besteht, ausgeführt wird.

11. Fläschchen nach einem der vorangehenden Ansprüche, **dadurch gekennzeichnet, dass** sie zum Verpacken einer Augenlösung ohne Konservierungsmittel verwendet wird.

## Claims

1. A bottle for packaging a liquid to be dispensed drop by drop comprising a reservoir for storing a preservative-free liquid, said reservoir having a wall with elastic deformation that is reversible by letting air into the reservoir by means of a dispensing head through which the liquid is delivered under the effect of a pressure exerted against said wall,
- wherein said head for dispensing the liquid comprises a nacelle (13) by which it is fitted in a sealed manner into the bottle, and a dropper tip (1) extending it to the outside of the bottle and which is pierced with a central channel (3) leading to an orifice for expulsion of the liquid,
and wherein an antibacterial filtering membrane (6), mounted on the base (4) of said tip, between the latter and said nacelle, is interposed across the flow of the air required to enter the bottle after a dispensing of liquid;
**characterized in that** selectively said dropper tip (1), situated beyond said antibacterial filtering membrane, to the exclusion of said nacelle (13), is made of a material containing a bactericidal agent having effect in the prevention of a bacterial proliferation on the surface of the material,
and **characterized in that** the tip (1)is made in two parts comprising a body (12) and a central core (7), said central core being inserted into the central channel of the tip and comprising a plurality of subchannels in order to define the circuit of expulsion of the liquid through said body, said body and said core both being made of materials containing bactericidal agents.

2. The bottle as claimed in claim 1, **characterized in that** it also comprises a pad for regulating the flow in said nacelle (13).

3. The bottle as claimed in claim 1 or 2, **characterized in that** the material of said body (12) contains a bactericidal agent distributed throughout its mass having effect by migration to the surface of the material while the central core (7) is made of a material comprising a bactericidal agent based on a chlorinated phenolic compound.

4. The bottle as claimed in claim 3, **characterized in that** said chlorinated phenolic compound in said core (7) is 5-chloro-2-(2,4-dichlorophenoxy)phenol, or triclosan.

5. The bottle as claimed in any one of claims, **characterized in that** said body (12) of the tip is made of a material containing as a bactericidal agent an ion-bearing polymer which is uniformly distributed throughout its mass.

6. The bottle as claimed in claim 5, **characterized in that** said bactericidal ions are silver ions.

7. The bottle as claimed in any one of claims, **characterized in that** the core (7) inside the tip (1) is a porous sintered thermoplastic material, in particular based on polyethylene.

8. The bottle as claimed in claim 7, **characterized in that** the porous material has a mean pore dimension, the order of magnitude of which is some hundred micrometers.

9. The bottle as claimed in one of claims 7 or 8, **characterized in that** the terminal portion of the expulsion channel of the external main body of the tip and the core made of porous material have a cylindrical shape, the cylindrical shape of the terminal portion of the expulsion channel conforming to the cylindrical shape of the core made of porous material.

10. The bottle as claimed in claim 9, **characterized in that** the body (12) of the tip is made by overmolding of the porous material constituting said core.

11. The bottle as claimed in any one of claims, **characterized in that** it is used for the packaging of an ophthalmic solution with no preservative.
